# EUROPÄISCHE PATENTSCHRIFT

(11) **EP 4 573 073 B1**
(45) Veröffentlichungstag und Bekanntmachung des Hinweises auf die Patenterteilung: **26.08.2026**
(21) Anmeldenummer: 23736715.6
(22) Anmeldetag: 30.06.2023
(51) Int. Cl.: C07C 5/25, C07C 7/04

(54) **VERFAHREN ZUR ISOMERISIERUNG VON OLEFINEN**
METHOD FOR ISOMERIFICATION OF OLEFINS
PROCÉDÉ D'ISOMÉRISATION DES OLÉFINES

(30) Priorität: 16.08.2022 EP 22190500
(43) Veröffentlichungstag der Anmeldung: 25.06.2025
(73) Patentinhaber: Evonik Oxeno GmbH & Co. KG, 45772 Marl (DE)
(72) Erfinder: MEIER, Ralf, 48324 Sendenhorst (DE); ZANTHOFF, Horst-Werner, 45481 Mülheim a.d. Ruhr (DE); METTERNICH, Jan Benedikt, 45657 Recklinghausen (DE); FLEISCHER, Vinzenz, 45770 Marl (DE)
(74) Vertreter: Evonik Patent Association
(86) Internationale Anmeldenummer: PCT/EP2023/068063
(87) Internationale Veröffentlichungsnummer: WO 2024/037771

(56) Entgegenhaltungen:
- EP-A1- 3 822 244
- EP-A2- 0 170 182
- WO-A1-2021/094162
- US-A- 5 177 281
- US-A1- 2005 070 747
- US-A1- 2018 057 423

## Beschreibung

Die vorliegende Erfindung betrifft ein Verfahren zur Isomerisierung von C4- bis C9-Olefinen mit endständiger Doppelbindung zu den entsprechenden Olefinen mit innenständiger Doppelbindung, wobei ein heterogener Katalysator eingesetzt wird, welcher eine Silicium-Aluminium-Mischoxidzusammensetzung umfasst.

Bei Isomerisierungen (bei Vorgängen innerhalb eines Moleküls spricht man auch von Umlagerungen) wird das Ausgangsmolekül in ein Molekül unveränderter Summenformel, aber geänderter Atomfolge, Atomanordnung oder Bindungsanordnungen überführt. Isomere besitzen oft vergleichbare Bindungsenergien, wodurch eine relativ freie Umwandlung ineinander stattfinden kann. Man unterscheidet, je nach Umwandlungsart z.B. die Bindungsisomerisierung, bei der Doppelbindungen beispielsweise zwischen C-C Bindungen umgelagert werden (wobei aber auch zahlreiche Bindungsisomerisierungen mit Beteiligung von Heteroatomen, wie O, N, P oder S dem Fachmann bekannt sind), die Skelettisomerisierung, bei der lineare Verbindungen in verzweigte umgelagert werden, die Hydroisomerisierung, bei der ein Alkan in Gegenwart von Wasserstoff über die Zwischenstufe eines Alkens in ein isomeres Alkan umgewandelt wird, oder die cis/trans Isomerisierung, bei der die Substituenten einer Doppelbindung umgelagert werden. Isomerisierungen werden häufig durch azide/basische Katalysatoren beschleunigt. Die Eigenschaften der Katalysatoren, wie die Stärke der Säure/Base-Zentren, entscheiden wesentlich, welche der Isomerisierungen in einem Molekül stattfinden. Die hier gewünschte Isomerisierung ist eine Bindungsisomerisierung.

Die entsprechenden Olefine mit endständiger oder innenständiger Doppelbindung können über verschiedene Verfahren, beispielsweise Crackingverfahren, bereitgestellt werden. Eine andere Möglichkeit ist die katalytische Isomerisierung von Olefinen mit endständiger Doppelbindung zu den entsprechenden Olefinen mit innenständiger Doppelbindung. Der Grad der Umwandlung ist jeweils durch das thermodynamische Gleichgewicht limitiert. Die katalytische Isomerisierung zu Olefinen mit endständiger Doppelbindung ist beispielsweise aus der EP 3 822 244 A1 bekannt.

US5177281A zeigt in den Beispielen 1-7 die Isomerisierung von 1-Buten mit einer Vielzahl von Katalysatoren in Kristallform wie ZSM-5, ZSM-22, ZSM-23, ZSM-34, ZSM-35 und ZSM-48. Das allgemeine Problem von Isomerisierungsreaktionen ist, dass die zu isomerisierenden Olefine aufgrund ihrer Doppelbindung reaktive Moleküle sind und deshalb Nebenreaktionen auftreten können. Ein Beispiel ist die Oligomerisierung, die an sauren Katalysatorsystem stattfinden kann und bei Einsatz entsprechend von sauren Katalysatoren bei der Isomerisierung als Nebenreaktion auftritt. Um ein Oligomerisieren der Olefine bei der Isomerisierung zu Olefinen mit endständiger Doppelbindung zu verhindern, werden eher basische Katalysatorsystem oder mit Alkali- oder Erdalkalimetallen dotierte Katalysatoren eingesetzt.

Nachteile an den bekannten Katalysatorsystemen war bislang, dass sie ausschließlich für die Isomerisierung von Olefinen mit innenständiger Doppelbindung zu Olefinen mit endständiger Doppelbindung beschrieben worden sind.

Die Aufgabe der vorliegenden Erfindung war deshalb ein Verfahren bereitzustellen, bei dem die Isomerisierung von Olefinen mit endständiger Doppelbindung zu Olefinen mit innenständiger Doppelbindung, insbesondere der Isomerisierung von 1-Buten zu 2-Buten, bevorzugt wird. Aus wirtschaftlicher Sicht sollte die Isomerisierung bei niedrigen Temperaturen, möglichst geringen Verweilzeiten, hohe Selektivität und einem hohen Umsatz erfolgen können. Der dabei eingesetzte Katalysator sollte zudem langzeitstabil sein und Nebenreaktionen wie die Oligomerisierung möglichst nicht begünstigen.

Überraschenderweise wurden gefunden, dass, abweichend davon, auch (schwach) saure, SiO₂-basierte Katalysatoren mit einem gewissen Aluminiumoxidanteil, also beispielsweise Katalysatoren basierend auf den hier genannten Silicium-Aluminium-Mischoxidzusammensetzungen, eingesetzt werden können, die bei einer guten Produktselektivität eine hohe Aktivität für die Isomerisierung liefern. Zudem tritt keine bzw. nahezu keine Oligomerisierung als Nebenreaktion auf.

Das erfindungsgemäße Verfahren ist demzufolge ein Verfahren zur Isomerisierung von C4- bis C9-Eduktolefinen mit endständiger Doppelbindung zu Produktolefinen mit innenständiger Doppelbindung, wobei eine Kohlenwasserstoffmischung, die zumindest die zu isomerisierenden Eduktolefine und Produktolefine enthält, mit einem heterogenen Katalysator in Kontakt gebracht wird, wobei der heterogene Katalysator eine röntgen-amorphe Silicium-Aluminium-Mischoxidzusammensetzung ist, die die nachfolgende Zusammensetzung aufweist:
a) 96 bis 99,99 Gew.-% Siliciumoxid (gerechnet als SiO₂); und
b) 0,01 bis 4 Gew.-% Aluminiumoxid (gerechnet als Al₂O₃).

Die als Katalysator eingesetzte röntgen-amorphe Silicium-Aluminium-Mischoxidzusammensetzung kann mittels Flammenhydrolyse nach dem unter anderem in der DE 198 47 161 A1 oder der EP 0 850 876 A1 offenbarten Verfahren hergestellt werden. Bei diesem sogenannten "co-fumed-Verfahren" werden flüchtige Silizium- und Aluminium-Verbindungen, z. B. Siliciumtetrachlorid und Aluminiumtrichlorid, in eine Knallgasflamme aus Wasserstoff und Sauerstoff bzw. Luft eingesprüht, wodurch die Silizium- und Aluminium-Verbindungen mittels des in der Knallgasflamme entstandenen Wassers hydrolysiert werden und sich die Mischoxidzusammensetzung bildet.

Ein alternatives Verfahren, welches ebenfalls in den genannten Schriften offenbart ist, ist das sogenannte Dotierverfahren. Dabei wird in der Knallgasflamme einerseits ein Oxid, hier beispielsweise Siliziumoxid, aus seiner flüchtigen Verbindung (z.B. Siliciumtetrachlorid) durch Flammenhydrolyse erzeugt und in die Knallgasflamme zusätzlich ein Aerosol eingespeist, in dem sich ein Salz des zu dotierenden Elementes, hier beispielsweise Aluminium, befindet und so das entsprechende Mischoxid bildet. Die so flammenhydrolytisch hergestellten Silicium-Aluminium-Mischoxidzusammensetzung ist überwiegend bis vollständig amorph.

Die mittels der exemplarisch genannten Herstellverfahren hergestellten röntgen-amorphen Silicium-Aluminium-Mischoxidzusammensetzung zeichnen sich durch ihre hohe chemische Reinheit aus und weisen die nachfolgende Zusammensetzung auf:
a) 96 bis 99,99 Gew.-% Siliciumoxid, vorzugsweise 98,5 bis 99,95 Gew.-% Siliciumoxid (gerechnet als SiO₂); und
b) 0,01 bis 4 Gew.-% Aluminimoxid, vorzugsweise 0,05 bis 1,5 Gew.-% Aluminiumoxid (gerechnet als Al₂O₃).

In einer bevorzugten Ausführungsform der vorliegenden Offenbarung enthält die Silicium-Aluminium-Mischoxidzusammensetzung zusätzlich Alkali- und/oder Erdalkalimetalloxide, besonders bevorzugt in einer Menge von bis zu 1 Gew.-% bezogen auf die Gesamtzusammensetzung. Um die Alkali- oder Erdalkalimetalloxide einzubringen, kann die flammenhydrolytisch hergestellte Mischoxidzusammensetzung mit einer wässrigen Alkalimetall- oder Erdalkalimetallhydroxid-Lösung behandelt werden. Das kann beispielsweise durch Tränken oder Imprägnieren des flammenhydrolytisch hergestellten Mischoxidzusammensetzung mit einer Alkali- und/oder Erdalkalimetallsalz-Lösung erfolgen. Anschließend wird die behandelte Mischoxidzusammensetzung mit Wasser gewaschen, bei 100 bis 150°C getrocknet und bei 300 bis 600°C, bevorzugt bei 450 bis 550°C, kalziniert. Silicium- und Aluminiumoxide können an sich auch bereits Spuren von Alkali- oder Erdalkalimetallen enthalten, die hier unberücksichtigt bleiben.

Die Silicium-Aluminium-Mischoxidzusammensetzungen der vorliegenden Erfindung können zusätzlich mit einer sauren, wässrigen Lösung mit einer Phosphorquelle behandelt werden. Als Phosphorquelle kann Phosphorsäure, Phosphonsäure, Phosphinsäure, Polyphosphorsäure oder Dihydrogenphosphat, bevorzugt Phosphorsäure, eingesetzt werden. Dazu wird die Mischoxidzusammensetzung zunächst in Wasser suspendiert wird und die entstandene Suspension anschließend mit der Phosphorquelle versetzt, vorzugsweise so, dass der pH-Wert im Bereich von 0 bis 6, weiterhin bevorzugt im Bereich von 1 bis 2.5, besonders bevorzugt im Bereich von 2 bis 2,5 liegt. Anschließend wird die behandelte Mischoxidzusammensetzung mit Wasser gewaschen, bei 100 bis 150°C getrocknet und bei 300 bis 600°C, bevorzugt bei 450 bis 550°C, kalziniert.

In einer bevorzugten Ausführungsform liegt die erfindungsgemäße Silicium-Aluminium-Mischoxidzusammensetzung überwiegend (d. h. > 70 %) oder vollständig in Form von aggregierten Primärpartikeln vor. Die Silicium-Aluminium-Mischoxidzusammensetzung zeichnet sich dabei unter anderem dadurch aus, dass das Gewichtsverhältnis (Al₂O₃/SiO₂)_{Oberfläche} der Primärpartikel im oberflächennahen Bereich kleiner ist als das Gewichtsverhältnis (Al₂O₃/SiO₂)_{gesamt} im gesamten Primärpartikel. Der Begriff "oberflächennaher Bereich" meint dabei den Bereich von der Oberfläche bis zu einer Tiefe von 5 nm. Der Unterschied in den Gewichtsverhältnissen bedeutet, dass die Aluminiumoxidkonzentration an der Oberfläche kleiner ist als in der gesamten Zusammensetzung. Der gesamte Primärpartikel schließt den Anteil an Siliciumdioxid und Aluminiumoxid im oberflächennahen Bereich mit ein.

Bevorzugt ist demnach eine Silicium-Aluminium-Mischoxidzusammensetzung, die überwiegend oder vollständig in Form von aggregierten Primärpartikeln vorliegt, bei denen
I) das Gewichtsverhältnis (Al₂O₃/SiO₂)_{gesamt} im Gesamtprimärpartikel 0,002 bis 0,05, bevorzugt 0,003 bis 0,015, besonders bevorzugt 0,005 bis 0,01 beträgt; und
II) das Gewichtsverhältnis (Al₂O₃/SiO₂)_{Oberfläche} der Primärpartikel im oberflächennahen Bereich kleiner ist als im Gesamtprimärpartikel.

Das Gewichtsverhältnis (Al₂O₃/SiO₂)_{Oberfläche} auf der Oberfläche kann zum Beispiel durch röntgeninduzierte Photoelektronen-Spektroskopie (XPS-Analyse) des Pulvers bestimmt werden. Zusätzliche Information über die Oberflächenzusammensetzung kann durch energiedispersive Röntgenstrahlung (TEM-EDX-Analyse) von einzelnen Primärpartikeln bestimmt werden. Das Gewichtsverhältnis (Al₂O₃/SiO₂)_{gesamt} im Gesamtprimärpartikel kann durch chemische oder physikalisch-chemische Methoden, z.B. Röntgenfluoreszenzanalyse, des Pulvers bestimmt werden

Die in der vorliegenden Erfindung als Katalysator verwendete Silicium-Aluminium-Mischoxidzusammensetzung ist röntgen-amorph. Röntgen-amorph im Sinne der vorliegenden Erfindung meint, dass eine röntgen-amorphe Substanz im Röntgendiffraktogramm bis zur Nachweisgrenze von 5 nm keine kristalline Struktur aufweist.

Die erfindungsgemäße beschriebene Silicium-Aluminium-Mischoxidzusammensetzung insbesondere mit der oben genannten Zusammensetzung und insbesondere mit den genannten Unterschieden in den Gewichtsverhältnissen (Al₂O₃/SiO₂) weist vorzugsweise eine BET-Oberfläche von 50 bis 250 m²/g, vorzugsweise 100 bis 200 m²/g auf (bestimmt gemäß DIN ISO 9277 ((Stand: 2014-01)).

Weiterhin kann es vorteilhaft sein, wenn die Silicium-Aluminium-Mischoxidzusammensetzung eine Dibutylphthalat-Zahl, in g Dibutylphthalat (DBP)/100 g Mischzusammensetzung, von 300 bis 350 aufweist. Die DBP-Zahl stellt ein Maß für die Struktur von Aggregaten dar. Niedrige Zahlen korrespondieren mit einer niedrigen Struktur, hohe Zahlen mit einer hohen Struktur. Der beschriebene Bereich von 300 bis 350 für die erfindungsgemäße Mischoxidzusammensetzung entspricht einer hohen Struktur. Bei der DBP-Absorption wird die Kraftaufnahme, beziehungsweise das Drehmoment (in Nm), der rotierenden Schaufeln des DBP-Messgerätes bei Zugabe definierter Mengen von DBP gemessen. Dabei ergibt sich für die Silicium-Aluminium-Mischoxidzusammensetzung vorzugsweise ein scharf ausgeprägtes Maximum mit einem anschließenden Abfall bei einer bestimmten Zugabe von DBP. Die Dibutylphthalatabsorption kann beispielsweise mit einem Gerät RHEOCORD 90 der Fa. Haake, Karlsruhe gemessen werden. Hierzu werden 12 g des Silicium-Aluminium-Mischoxidpulvers in eine Knetkammer eingefüllt, diese mit einem Deckel verschlossen und Dibutylphthalat über ein Loch im Deckel mit einer vorgegebenen Dosierrate von 0,0667 ml/s eindosiert. Der Kneter wird mit einer Motordrehzahl von 125 Umdrehungen pro Minute betrieben. Nach Erreichen des Drehmomentmaximums wird der Kneter und die DBP-Dosierung automatisch abgeschaltet. Aus der verbrauchten Menge DBP und der eingewogenen Menge der Partikel wird die DBP-Absorption berechnet nach: DBP-Zahl (g/100 g) = (Verbrauch DBP in g / Einwaage Pulver in g) x 100.

Für die industriell betriebene Isomerisierung unter Verwendung eines Katalysators, der die Silicium-Aluminium-Mischoxidzusammensetzung umfasst, ist eine Reaktionsführung in einem oder mehreren Festbettreaktoren bevorzugt. Für Flüssigphasenreaktionen können auch Slurry-Reaktoren oder Trickle-Bed-Reaktoren eingesetzt werden. Es können auch andere Reaktortypen, wie Wirbelbettreaktoren oder Wanderbettreaktoren, eingesetzt werden. Dazu ist es erforderlich, dass die zuvor beschriebene, flammhydrolytisch bzw. pyrogen hergestellte Mischoxidzusammensetzung unter Zugabe eines Bindemittels mittels eines dem Fachmann bekannten formgebenden Verfahrens in Form gebracht wird, insbesondere in Form von Granulaten, Pellets oder Formkörpern, wie Tabletten, Zylindern, Kugeln, Strangextrudaten oder Ringen. Geeignete Bindemittel sind dem Fachmann bekannt, beispielswiese Tonerde, keramische Tonen, Kolloiden oder auch amorphe Zeolithe Verwendung finden.

Zur Formgebung werden zunächst 1 bis 20 Gew.-% der Silicium-Aluminium-Mischoxidzusammensetzung mit einem der vorgenannten Bindemittel und zusätzlich mit temporären Hilfsstoffen, wie beispielsweise Wasser, wässrigen Lösungen, Wasserersatzstoffen, wie Glykolen oder Polyglykolen, und optional weiteren Hilfsstoffen, wie Fixierungsmitteln, beispielsweise Celluloseethern, und/oder Plastifizierungsmitteln, beispielsweise Polysacchariden, und/oder Presshilfsmitteln, beispielsweise nichtionischen Wachsdispersionen, vermischt. Dieser Vorgang kann in dem Fachmann bekannten Vorrichtungen, beispielsweise in einem Kneter oder einem Intensivmischer, durchgeführt werden. Anschließend findet die eigentliche Formgebung durch ein formgebendes Verfahren, wie beispielsweise Pelletierung, Extrusion oder Trockenpressen, statt. Vor dem Einbau in den oder die Festbettreaktoren werden die Formen bzw. Formkörper in einem Temperaturbereich von 200 bis 700°C kalziniert, wodurch zumindest die temporären Hilfsstoffe entfernt werden.

Die Silicium-Aluminium-Mischoxidzusammensetzung kann auf einem für die Isomerisierung inerten Träger, beispielsweise einem Metall-, Kunststoff- oder Keramikträger, aufgebracht werden. Ist die Silicium-Aluminium-Mischoxidzusammensetzung auf einem inerten Träger aufgebracht, wird die Masse und Zusammensetzung des inerten Trägers bei der Bestimmung der Zusammensetzung der Silicium-Aluminium-Mischoxidzusammensetzung nicht berücksichtigt.

Das erfindungsgemäße Verfahren wird mit der vorgenannt beschriebenen Silicium-Aluminium-Mischoxidzusammensetzung als Katalysator durchgeführt, um C4- bis C9-Eduktolefinen mit endständiger Doppelbindung, vorzugsweise C4- bis C8-Eduktolefinen mit endständige Doppelbindung, weiterhin bevorzugt C4- bis C6-Eduktolefinen mit endständige Doppelbindung, besonders bevorzugt C4-Eduktolefinen mit endständige Doppelbindung, zu Produktolefinen mit innenständiger Doppelbindung zu isomerisieren.

Die Olefine werden nicht unbedingt in reiner Form eingesetzt, sondern in technisch verfügbaren Kohlenwasserstoffmischungen. Durch die Isomerisierung wird der Gehalt des Produktolefins demzufolge in der Kohlenwasserstoffmischung erhöht und gleichzeitig der Gehalt an Eduktolefin verringert.

C5-Olefine sind in Leichtbenzinfraktionen aus Raffinerien oder Crackern enthalten. Technische Gemische, die lineare C4-Olefine enthalten, sind Leichtbenzinfraktionen aus Raffinerien, C4-Fraktionen aus FC- oder Steamcrackern, Gemische aus Fischer-Tropsch-Synthesen, Gemische aus der Dehydrierung von Butanen und durch Metathese oder aus anderen technischen Prozessen entstandene Gemische.

Bevorzugt können für das erfindungsgemäße Verfahren geeignete Gemische linearer Butene aus der C4-Fraktion eines Steamcrackers gewonnen werden. Dabei kann in einem Vorschritt 1,3 Butadien entfernt werden. Dies geschieht entweder durch Extraktion(sdestillation) des Butadiens oder dessen Selektivhydrierung. In beiden Fällen wird ein praktisch butadienfreier C4-Schnitt erhalten, das sogenannte Raffinat I. Der jetzt Butadien- freie C4-Schnitt, das sogenannte Raffinat I, enthält iso-Buten, die linearen Butene und gegebenenfalls Butane.

Die Eduktolefine sind erfindungsgemäß Olefine mit endständiger Doppelbindung, die durch die Isomerisierung zumindest teilweise in Produktolefine, also Olefine mit innenständiger Doppelbindung ist, umgewandelt werden. In einer bevorzugten Ausführungsform handelt es sich beim Eduktolefin um 1-Buten bzw. Kohlenwasserstoffmischungen, die 1-Buten enthalten, das durch die erfindungsgemäße Isomerisierung in cis- und/oder trans-2-Buten umgewandelt wird. Das ermöglicht die Anreicherung von 2-Butenen, um die destillative Abtrennung von Isobuten zu erleichtern. Das hat den Vorteil, dass Energie eingespart werden und ein signifikant größerer Durchsatz bei gleichen Apparaten erreicht werden kann. 2-Buten kann anschließend zu C8-Olefinen oligomerisiert werden, woraus wiederum Weichmacheralkohole gemacht werden können. Der Umsatz des Eduktolefins zum Produktolefin ist insbesondere durch die temperaturabhängige Lage des chemischen Gleichgewichts der Isomerisierungsreaktion nach unten begrenzt. Der Vorteil durch die Verwendung eines erfindungsgemäßen Katalysators ist, dass der Umsatz dem thermodynamischen Gleichgewichtsumsatz in einem breiteren Temperaturbereich entspricht oder diesen nur geringfügig unterschreitet. Dies gilt auch im Hinblick auf die Isomerisierung von 1-Buten zu 2-Buten, die durch das thermodynamische Gleichgewicht der n-Buten-Isomere begrenzt ist. Das thermodynamische Gleichgewicht eines 2-Butene und 1-Buten enthaltenen Gemisches wird durch hohe Temperaturen in Richtung des 1-Butens verschoben. Das thermodynamische Gleichgewicht für 1-Buten liegt bei einer Temperatur von 25 °C bei etwa 3 % und bei einer Temperatur von 500 °C bei etwa 29 %.

Für das erfindungsgemäße Isomerisierungsverfahren wird vorzugsweise mindestens ein Festbettreaktor eingesetzt. Es können auch andere Reaktortypen, wie Wirbelbettreaktoren, Wanderbettreaktoren, Slurry-Reaktoren oder Trickle-Bed-Reaktoren eingesetzt werden.

Das Verfahren gemäß dieser Offenbarung kann bei Atmosphärendruck durchgeführt werden. Es können jedoch auch höhere Reaktionsdrücke angewandt werden. Die Druckfahrweise im Verfahren gemäß der vorliegenden Offenbarung ist beispielsweise sinnvoll, wenn das Produktolefin aus dem Isomerisierungsverfahren gemäß der vorliegenden Offenbarung noch einer Trennstufe zugeführt wird, die ebenfalls unter Druck betrieben wird.

Die erfindungsgemäße Isomerisierung Olefinen mit endständiger Doppelbindung zu Olefinen mit innenständiger Doppelbindung, insbesondere von 1-Buten zu 2-Buten, erfolgt vorzugsweise bei einer Temperatur zwischen 20 °C und 250 °C, weiterhin bevorzugt zwischen 35 °C und 200 °C und besonders bevorzugt zwischen 45 °C und 160 °C. Die Gas-Raumgeschwindigkeiten (gas hourly space velocity = GHSV) können von 5 bis 500 h⁻¹, vorzugsweise von 10 bis 250 h⁻¹ betragen. Die Selektivität der erfindungsgemäßen Isomerisierung bezüglich des Produktolefins ist vorzugsweise größer als 85%, weiterhin bevorzugt größer als 90% und besonders bevorzugt größer als oder gleich 95% ist.

Bei Nachlassen der Aktivität und Selektivität des erfindungsgemäßen Katalysators infolge von Kohlenstoffablagerungen auf dem Katalysator wird dieser zweckmäßigerweise regeneriert. Ein vorteilhaftes Verfahren zur Katalysatorregenerierung besteht darin, dass man die Kohlenstoffablagerungen auf dem desaktivierten Katalysator in Sauerstoff enthaltenden Gasen, vorzugsweise in Luft, abbrennt. Es kann zweckmäßig sein, hierbei die Luft mit Stickstoff zu verdünnen. Die Katalysatorregenerierung wird im Allgemeinen bei Temperaturen von 350 bis 600 °C, vorzugsweise von 400 bis 450 °C, durchgeführt. Hierdurch lassen sich in der Regel die Anfangsaktivität und die Anfangsselektivität des erfindungsgemäßen Katalysators in einfacher Weise zurückgewinnen.

Gegenstand der vorliegenden Erfindung ist weiterhin ein Verfahren zur destillativen Abtrennung von Isobuten aus C4-Kohlenwasserstoffströmen, die zumindest Isobutene, 1-Butene und 2-Butene umfassen, wobei das Verfahren die folgenden Schritte umfasst:
1) Durchführung einer Isomerisierung mit dem C4-Kohlenwasserstoffstrom, wodurch die 1-Butene in dem C4-Kohlenwasserstoffstrom zumindest teilweise zu 2-Butenen umgewandelt werden, wobei der C4-Kohlenwasserstoffstrom mit einem heterogenen Katalysator in Kontakt gebracht wird, wobei der heterogene Katalysator eine Silicium-Aluminium-Mischoxidzusammensetzung ist, die die nachfolgende Zusammensetzung aufweist:
   a) 96 bis 99,99 Gew.-%, vorzugsweise 98,5 bis 99,95 Gew.-% Siliciumoxid (gerechnet als SiO₂); und
   b) 0,01 bis 4 Gew.-%, vorzugsweise 0,05 bis 1,5 Gew.-% Aluminiumoxid (gerechnet als Al₂O₃); und
2) Durchführung einer destillativen Abtrennung, um Isobuten aus dem C4-Kohlenwasserstoffstrom abzutrennen.

Die destillative Abtrennung von Isobuten aus dem C4-Kohlenwasserstoffstrom ist dem Fachmann grundsätzlich bekannt. Die Destillation in Schritt 2) wird vorzugsweise bei einem Druck von 1 - 10 bar, vorzugsweise von 2 bis 8 bar durchgeführt. Die Temperatur bei der Destillation in Schritt 2) beträgt vorzugsweise 20 bis 80 °C, besonders bevorzugt 25 bis 70 °C. Die Destillation in Schritt 2) kann weiterhin mit bekannten Destillationskolonnen durchgeführt werden. Die Kolonne kann eine Vielzahl von Böden und/oder Trennstufen umfassen.

Gegenstand der vorliegenden Erfindung ist weiterhin eine zweistufige Isomerisierung mit zwischengeschalteter Destillation. In der ersten Stufe wird die vorgenannte beschriebene Isomerisierung durchgeführt, also eine Isomerisierung von C4- bis C9-Eduktolefinen, vorzugsweise C4-Olefinen, mit endständiger Doppelbindung, vorzugsweise 1-Butene, zu Produktolefinen mit innenständiger Doppelbindung, vorzugsweise 2-Butene, wobei eine Kohlenwasserstoffmischung, die zumindest die zu isomerisierenden Eduktolefine und Produktolefine enthält, mit einem heterogenen Katalysator in Kontakt gebracht wird, wobei der heterogene Katalysator eine Silicium-Aluminium-Mischoxidzusammensetzung ist, die die nachfolgende Zusammensetzung aufweist:
a) 96 bis 99,99 Gew.-%, vorzugsweise 98,5 bis 99,95 Gew.-% Siliciumoxid (gerechnet als SiO₂); und
b) 0,01 bis 4 Gew.-%, vorzugsweise 0,05 bis 1,5 Gew.-% Aluminiumoxid (gerechnet als Al₂O₃).

Nach der Isomerisierung erfolgt eine Destillation, bei der die abgetrennten Eduktolefine mit endständiger Doppelbindung, vorzugsweise die 1-Butene zusammen mit dem Isobutenen, von den Produktolefinen mit innenständiger Doppelbindung, vorzugsweise den 2-Butenen, abgetrennt. Entsprechende Verfahren und die jeweiligen Bedingungen sind dem Fachmann geläufig.

Die abgetrennten Eduktolefine, vorzugsweise die aus der Abtrennung erhaltene Mischung aus 1-Butenen und Isobutenen, werden dann in einem zweiten Schritt einer weiteren Isomerisierung unterworfen. Als Katalysator kann die auch im ersten Schritt verwendete Silicium-Aluminium-Mischoxidzusammensetzung eingesetzt werden, die die nachfolgende Zusammensetzung aufweist:
a) 96 bis 99,99 Gew.-%, vorzugsweise 98,5 bis 99,95 Gew.-% Siliciumoxid (gerechnet als SiO₂); und
b) 0,01 bis 4 Gew.-%, vorzugsweise 0,05 bis 1,5 Gew.-% Aluminiumoxid (gerechnet als Al₂O₃).

Im zweiten Schritt können neben der Isomerisierung auch andere Reaktionen auftreten, beispielsweise Dimerisierungen der enthaltenen Olefine. Bei C4-Olefinen können insbesondere die Isobutene zu Di-Isobutenen dimerisieren, die dann aber auch recht einfach abgetrennt und vermarktet werden können.

Die Erfindung wird nachfolgend anhand eines Beispiels beschrieben. Dies dient der Erläuterung und stellt keine Einschränkung des Erfindungsgegenstands dar.

### Beispiel 1

11.7 g eines erfindungsgemäßen Katalysators (AEROSIL^{®} MOX170, ca. 1 Gew.-% Aluminiumoxid, BET Oberfläche zwischen 140 und 200 m²/g) wurden in einem Rohrreaktor mit einem Durchmesser von 1 cm nach 1:1 Verdünnung mit Glasperlen eingefüllt. Der Reaktor wurde mit 1-Buten (>99%) beschickt. Dabei wurde das 1-Buten mit unterschiedlichen Volumenströmen durch den Reaktor geleitet. Die Isomerisierung erfolgte bei Temperaturen von 80 bis 140 °C und Umgebungsdruck. Bei der Reaktion wurden die Umsätze von 1-Buten und die Bildung von 2-Buten bestimmt. Die Analyse erfolgte durch Gas-Chromatographie. Ausgewertet wurden die Peak-Flächen nach Methode der externen Kalibration.

| Vol.-Strom 1-Buten [ml/h] | Temperatur [°C] | Umsatz 1-Buten [%] | Selektivität Bildung 2-Buten [%] |
|---|---|---|---|
| 250 | 80 | 90.2 | 98.1 |
| 250 | 140 | 89.5 | 94.9 |
| 420 | 80 | 88.9^{a} | 98.2^{a} |

| | | | |
|---|---|---|---|
| ^{a}Über 500 Stunden Versuchszeit. | | | |

Die Ergebnisse zeigen, dass die erfindungsgemäßen Katalysatoren sehr gut für die Isomerisierung geeignet sind. Es ist weiterhin zu erkennen, dass kaum Nebenreaktionen auftreten, sondern hohe Selektivtäten zu 2-Buten erreicht werden können. Das gilt auch für Langzeitversuche mit mehr als 500 Stunden Versuchsdauer.

## Patentansprüche

1. Verfahren zur Isomerisierung von C4- bis C9-Eduktolefinen mit endständiger Doppelbindung zu Produktolefinen mit innenständiger Doppelbindung, wobei eine Kohlenwasserstoffmischung, die zumindest die zu isomerisierenden Eduktolefine und Produktolefine enthält, mit einem heterogenen Katalysator in Kontakt gebracht wird, wobei der heterogene Katalysator eine röntgen-amorphe Silicium-Aluminium-Mischoxidzusammensetzung ist, die die nachfolgende Zusammensetzung aufweist:
a) 96 bis 99,99 Gew.-% Siliciumoxid (gerechnet als SiO₂); und
b) 0,01 bis 4 Gew.-% Aluminiumoxid (gerechnet als Al₂O₃).

2. Verfahren nach Anspruch 1, wobei die als heterogener Katalysator eingesetzte Silicium-Aluminium-Mischoxidzusammensetzung die folgende Zusammensetzung aufweist:
a) 98,5 bis 99,95 Gew.-% Siliciumoxid (gerechnet als SiO₂); und
b) 0,05 bis 1,5 Gew.-% Aluminiumoxid (gerechnet als Al₂O₃).

3. Verfahren nach Anspruch 1 oder 2, wobei die als heterogener Katalysator eingesetzte Silicium-Aluminium-Mischoxidzusammensetzung eine BET-Oberfläche von 50 bis 250 m²/g, vorzugsweise 100 bis 220 m²/g aufweist.

4. Verfahren nach einem der Ansprüche 1 bis 3, wobei der heterogene Katalysator aus Formkörpern besteht, die aus der Silicium-Aluminium-Mischoxidzusammensetzung unter Zugabe von Bindemitteln und zumindest temporären Hilfsstoffen in einem formgebenden Verfahren hergestellt wurden.

5. Verfahren nach einem der Ansprüche 1 bis 4, wobei der Gehalt des Produktolefins in der Kohlenwasserstoffmischung durch die Isomerisierung erhöht wird.

6. Verfahren nach einem der Ansprüche 1 bis 5, wobei die Isomerisierung bei einer Temperatur zwischen 20 °C und 250 °C, weiterhin bevorzugt zwischen 35 °C und 200 °C und besonders bevorzugt zwischen 45 °C und 160 °C durchgeführt wird.

7. Verfahren nach einem der Ansprüche 1 bis 6, wobei die Gas-Raumgeschwindigkeit bei der Isomerisierung von 5 bis 500 h⁻¹, vorzugsweise von 10 bis 250 h⁻¹ beträgt.

8. Verfahren einem der Ansprüche 1 bis 7, wobei als Eduktolefine C4- bis C8-Eduktolefine mit endständiger Doppelbindung, vorzugsweise C4- bis C6-Eduktolefinen mit endständiger Doppelbindung, besonders bevorzugt C4-Eduktolefinen mit endständiger Doppelbindung eingesetzt werden.

9. Verfahren nach Anspruch 8, wobei als Eduktolefine 1-Buten bzw.
Kohlenwasserstoffmischungen, die 1-Buten enthalten, eingesetzt werden und das Produktolefin mit innenständiger Doppelbindung cis- und/oder trans-2-Buten ist.

10. Verfahren nach einem der Ansprüche 1 bis 9, wobei die Silicium-Aluminium-Mischoxidzusammensetzung überwiegend oder vollständig in Form von aggregierten Primärpartikeln vorliegt.

11. Verfahren nach Anspruch 10, wobei die Silicium-Aluminium-Mischoxidzusammensetzung sich dadurch auszeichnet, dass das Gewichtsverhältnis (Al₂O₃/SiO₂)_{Oberfläche} der Primärpartikel im oberflächennahen Bereich kleiner ist als das Gewichtsverhältnis (Al₂O₃/SiO₂)_{gesamt} im gesamten Primärpartikel.

12. Verfahren nach Anspruch 10 oder 11, wobei die Silicium-Aluminium-Mischoxidzusammensetzung überwiegend oder vollständig in Form von aggregierten Primärpartikeln vorliegt, bei denen
I) das Gewichtsverhältnis von (Al₂O₃/SiO₂)_{gesamt} im Gesamtprimärpartikel 0,002 bis 0,05, bevorzugt 0,003 bis 0,015, besonders bevorzugt 0,005 bis 0,01 beträgt; und
II) das Gewichtsverhältnis (Al₂O₃/SiO₂)_{Oberfläche} der Primärpartikel in einer oberflächennahen Schicht mit einer Dicke von 5 nm kleiner als im Gesamtprimärpartikel ist.

13. Verfahren zur destillativen Abtrennung von Isobuten aus C4-Kohlenwasserstoffströmen, die zumindest Isobutene, 1-Butene und 2-Butene umfassen, wobei das Verfahren die folgenden Schritte umfasst:
1) Durchführung einer Isomerisierung nach einem der Ansprüche 1 bis 13, wodurch die 1-Butene in dem C4-Kohlenwasserstoffstrom zumindest teilweise zu 2-Butenen umgewandelt werden;
2) Durchführung einer destillativen Abtrennung, um Isobuten und 1-Buten aus dem C4-Kohlenwasserstoffstrom abzutrennen.

14. Verfahren nach Anspruch 13, wobei die Isomerisierung zweistufig durchgeführt wird, wobei nach der Destillation in Schritt 2) die aus der Abtrennung erhaltene Mischung aus 1-Butenen und Isobutenen in einem zweiten Schritt einer weiteren Isomerisierung unterworfen werden.

## Claims

1. Process for the isomerization of C4 to C9 reactant olefins having a terminal double bond to product olefins having an internal double bond, wherein a hydrocarbon mixture comprising at least the reactant olefins to be isomerized and product olefins is brought into contact with a heterogeneous catalyst, wherein the heterogeneous catalyst is an X-ray-amorphous silicon-aluminium mixed oxide composition having the following composition:
a) 96% to 99.99% by weight of silicon oxide (calculated as SiO₂); and
b) 0.01% to 4% by weight of aluminium oxide (calculated as Al₂O₃).

2. Process according to Claim 1, wherein the silicon-aluminium mixed oxide composition used as heterogeneous catalyst has the following composition:
a) 98.5% to 99.95% by weight of silicon oxide (calculated as SiO₂); and
b) 0.05% to 1.5% by weight of aluminium oxide (calculated as Al₂O₃).

3. Process according to Claim 1 or 2, wherein the silicon-aluminium mixed oxide composition used as heterogeneous catalyst has a BET surface area of 50 to 250 m²/g, preferably 100 to 220 m²/g.

4. Process according to any of Claims 1 or 3, wherein the heterogeneous catalyst consists of shaped bodies produced from the silicon-aluminium mixed oxide composition in a shaping process with addition of binders and at least temporary auxiliaries.

5. Process according to any of Claims 1 to 4, wherein the content of the product olefin in the hydrocarbon mixture is increased as a result of the isomerization.

6. Process according to any of Claims 1 to 5, wherein the isomerization is carried out at a temperature between 20°C and 250°C, further preferably between 35°C and 200°C and particularly preferably between 45°C and 160°C.

7. Process according to any of Claims 1 to 6, wherein the gas hourly space velocity during the isomerization is from 5 to 500 h⁻¹, preferably from 10 to 250 h⁻¹.

8. Process according to any of Claims 1 to 7, wherein the reactant olefins used are C4 to C8 reactant olefins having a terminal double bond, preferably C4 to C6 reactant olefins having a terminal double bond, particularly preferably C4 reactant olefins having a terminal double bond.

9. Process according to Claim 8, wherein the reactant olefins used are 1-butene or hydrocarbon mixtures comprising 1-butene, and the product olefin having an internal double bond is cis- and/or trans-2-butene.

10. Process according to any of Claims 1 to 9, wherein the silicon-aluminium mixed oxide composition is predominantly or entirely present in the form of aggregated primary particles.

11. Process according to Claim 10, wherein the silicon-aluminium mixed oxide composition has the feature that the weight ratio (Al₂O₃/SiO₂)_{surface} of the primary particles in the near-surface region is less than the weight ratio (Al₂O₃/SiO₂)ₜₒₜₐₗ in the totality of the primary particles.

12. Process according to Claim 10 or 11, wherein the silicon-aluminium mixed oxide composition is predominantly or entirely present in the form of aggregated primary particles in which
I) the weight ratio of (Al₂O₃/SiO₂) ₜₒₜₐₗ in the totality of the primary particles is 0.002 to 0.05, preferably 0.003 to 0.015, particularly preferably 0.005 to 0.01; and
II) the weight ratio (Al₂O₃/SiO₂)_{surface} of the primary particles in a near-surface layer having a thickness of 5 nm is less than in the totality of the primary particles.

13. Process for the distillative removal of isobutene from C4 hydrocarbon streams comprising at least isobutenes, 1-butenes and 2-butenes, wherein the process comprises the following steps:
1) carrying out an isomerization according to any of Claims 1 to 13, whereby the 1-butenes in the C4 hydrocarbon stream are at least partially converted into 2-butenes;
2) carrying out a distillative removal in order to remove isobutene and 1-butene from the C4 hydrocarbon stream.

14. Process according to Claim 13, wherein the isomerization is carried out in two stages, wherein after the distillation in step 2) the mixture of 1-butenes and isobutenes obtained from the removal is subjected in a second step to a further isomerization.

## Revendications

1. Procédé d'isomérisation d'oléfines de départ en C4 à C9 présentant une double liaison terminale en oléfines produits présentant une double liaison interne, un mélange d'hydrocarbures contenant au moins les oléfines de départ à isomériser et les oléfines produits étant mis en contact avec un catalyseur hétérogène, le catalyseur hétérogène étant une composition d'oxyde mixte de silicium-aluminium amorphe aux rayons X présentant la composition suivante :
a) 96 à 99,99 % en poids d'oxyde de silicium (calculé sous forme de SiO₂) ; et
b) 0,01 à 4 % en poids d'oxyde d'aluminium (calculé sous forme d'Al₂O₃).

2. Procédé selon la revendication 1, la composition d'oxyde mixte de silicium-aluminium utilisée en tant que catalyseur hétérogène présentant la composition suivante :
a) 98,5 à 99,95 % en poids d'oxyde de silicium (calculé sous forme de SiO₂) ; et
b) 0,05 à 1,5 % en poids d'oxyde d'aluminium (calculé sous forme d'Al₂O₃).

3. Procédé selon la revendication 1 ou 2, la composition d'oxyde mixte de silicium-aluminium utilisée en tant que catalyseur hétérogène présentant une surface BET de 50 à 250 m²/g, de préférence de 100 à 220 m²/g.

4. Procédé selon l'une quelconque des revendications 1 à 3, le catalyseur hétérogène étant constitué de corps moulés qui ont été fabriqués à partir de la composition d'oxyde mixte de silicium-aluminium par un procédé de mise en forme avec ajout de liants et d'auxiliaires au moins temporaires.

5. Procédé selon l'une quelconque des revendications 1 à 4, la teneur de l'oléfine produit dans le mélange d'hydrocarbures étant augmentée par l'isomérisation.

6. Procédé selon l'une quelconque des revendications 1 à 5, l'isomérisation étant réalisée à une température comprise entre 20 °C et 250 °C, plus préférablement entre 35 °C et 200 °C et particulièrement préférablement entre 45 °C et 160 °C.

7. Procédé selon l'une quelconque des revendications 1 à 6, la vitesse spatiale gazeuse lors de l'isomérisation étant de 5 à 500 h⁻¹, de préférence de 10 à 250 h⁻¹.

8. Procédé selon l'une quelconque des revendications 1 à 7, des oléfines de départ en C4 à C8 présentant une double liaison terminale, de préférence des oléfines de départ en C4 à C6 présentant une double liaison terminale, et de manière particulièrement préférée des oléfines de départ en C4 présentant une double liaison terminale, étant utilisées en tant qu'oléfines de départ.

9. Procédé selon la revendication 8, du 1-butène ou des mélanges d'hydrocarbures contenant du 1-butène étant utilisés comme oléfines de départ, et l'oléfine produit présentant une double liaison interne étant du cis- et/ou du trans-2-butène.

10. Procédé selon l'une quelconque des revendications 1 à 9, la composition d'oxyde mixte de silicium-aluminium étant présente majoritairement ou entièrement sous forme de particules primaires agrégées.

11. Procédé selon la revendication 10, la composition d'oxyde mixte de silicium-aluminium **se caractérisant en ce que** le rapport pondéral (Al₂O₃/SiO₂) _{surface} des particules primaires dans la région proche de la surface est inférieur au rapport pondéral (Al₂O₃/SiO₂) ₜₒₜₐₗ dans l'ensemble de la particule primaire.

12. Procédé selon la revendication 10 ou 11, la composition d'oxyde mixte de silicium-aluminium étant présente majoritairement ou entièrement sous forme de particules primaires agrégées, dans lesquelles
I) le rapport pondéral de (Al₂O₃/SiO₂) ₜₒₜₐₗ dans l'ensemble de la particule primaire est de 0,002 à 0,05, de préférence de 0,003 à 0,015, de manière particulièrement préférée de 0,005 à 0,01 ; et
II) le rapport pondéral (Al₂O₃/SiO₂)_{surfacee} des particules primaires dans une couche proche de la surface présentant une épaisseur de 5 nm est inférieur à celui dans l'ensemble de la particule primaire.

13. Procédé de séparation distillative d'isobutène à partir de flux d'hydrocarbures en C4 comprenant au moins des isobutènes, des 1-butènes et des 2-butènes, le procédé comprenant les étapes suivantes :
1) la réalisation d'une isomérisation selon l'une quelconque des revendications 1 à 13, par laquelle les 1-butènes dans le flux d'hydrocarbures en C4 sont convertis au moins partiellement en 2-butènes ;
2) la réalisation d'une séparation distillative afin de séparer l'isobutène et le 1-butène du flux d'hydrocarbures en C4.

14. Procédé selon la revendication 13, l'isomérisation étant réalisée en deux étapes, la séparation distillative de l'étape 2) étant suivie de la soumission du mélange de 1-butènes et d'isobutènes obtenu à partir de la séparation à une autre isomérisation lors d'une deuxième étape.
